# EUROPÄISCHE PATENTSCHRIFT

(11) **EP 0 927 713 B1**
(45) Veröffentlichungstag und Bekanntmachung des Hinweises auf die Patenterteilung: **22.08.2001**
(21) Anmeldenummer: 98124510.3
(22) Anmeldetag: 22.12.1998
(51) Int. Cl.: C07C 67/343, C07C 67/303, C07C 69/716, C07C 69/738

(54) **Verbessertes Verfahren zur Herstellung von 2-Acetylcarbonsäureestern**
Process for the preparation of esters of 2-acetylcarboxylic acids
Procédé de préparation d'esters d'acides 2-acétylcarboxyliques

(30) Priorität: 29.12.1997 AT 219397
(43) Veröffentlichungstag der Anmeldung: 07.07.1999
(73) Patentinhaber: DSM Fine Chemicals Austria Nfg GmbH & Co KG, 4021 Linz (AT)
(72) Erfinder: Huber, Wolfgang, Dr., 5020 Salzburg (AT); Eichberger, Günter, Dr., 4616 Weisskirchen (AT); Pöschko, Harald, 4432 Ennsdorf (AT); Burschik, Gerhard, 4030 Linz (AT)
(74) Vertreter: Klostermann, Ingrid

(56) Entgegenhaltungen:
- EP-A- 0 100 019
- FR-A- 2 073 109

## Beschreibung

Acetylcarbonsäureester werden für die Herstellung einer Vielzahl von unterschiedlichsten Produkten, wie etwa Fungizide oder insektizide Pyrimidinverbindungen Pankreaslipaseinhibitoren oder Parfumprodukte, verwendet.

In der Literatur sind bereits mehrere Synthesewege zur Herstellung von Acetylcarbonsäureestem beschrieben. Eine mögliche Variante ist die Umsetzung von Acetessigsäureestern mit einem Alkylbromid oder -jodid (z.B. EP-A-0 443 449). Bei dieser Variante entstehen jedoch leicht dialkylierte Verbindungen oder O-Alkylacetatessigsäureester, die sich destillativ nicht von gewünschten Endprodukten abtrennen lassen. Eine alternative Methode stellt, wie etwa in FR-A-2 073 109 bzw. DE 2 060 443 im Stand der Technik zitiert, eine Eintopfreaktion dar, gemäß welcher der Acetessigsäureester mit einem Aldehyd kondensiert wird und das so erhaltene Reaktionsgemisch, ohne Isolierung des gebildeten Zwischenproduktes, zum gewünschten Endprodukt hydriert wird. Bei dieser Reaktion entstehen jedoch eine Vielzahl von Nebenverbindungen, wie etwa Kondensationsprodukte u.s.w. Weiters ist die Ausbeute an Acetylcarbonsäureester unbefriedigend. (<40 %).

Dies gilt auch für das in DE 20 60 443 beschriebene Verfahren, gemäß welchem Alkylacylessigsäureester durch Hydrieren einer Mischung aus einem Acylessigsäureester, einem Aldehyd und einem Kondensationskatalysator in Gegenwart eines Hydrierkatalysators hergestellt werden.
Auch in EP 0 795 538 ist darauf hingewiesen, daß dieses Verfahren zu Endprodukten mit schwierig abtrennbaren Alkenylacetessigsäureestern führt.

Aufgabe der Erfindung war es deshalb ein verbessertes Verfahren zur Herstellung von 2-Acetylcarbonsäureestern zu finden, das den gewünschten Ester in hohen Ausbeuten und in hoher Reinheit, unter Vermeidung der Bildung von Kondensationsnebenprodukten, liefert.

Gegenstand der Erfindung ist demnach ein verbessertes Verfahren zur Herstellung von 2-Acetylcarbonsaureester durch Reaktion eines Acetessigsäureesters mit einem Aldehyd und anschließender Hydrierung, das dadurch gekennzeichnet ist, daß
a) ein Acetessigsäureester mit 1 bis 6 C-Atomen im Alkylteil mit einem aliphatischen Aldehyd mit 1 bis 12 C-Atomen in Gegenwart eines Kondensationskatalysators bei 0 bis 40°C umgesetzt wird,
b) nach erfolgter Kondensation das gebildete Reaktionswasser, sowie nicht umgesetzte Edukte aus dem Reaktionsgemisch entfernt werden, anschließend
c) der verbleibende Rückstand in Gegenwart eines Hydrierkatalysators in Substanz bei 20 bis 160°C und einem Druck von 1 bis 100 bar hydriert wird, wobei in der ersten Phase des Hydrierens die Temperatur zwischen 20 und 90°C liegt und in der zweiten Phase die Temperatur, je nach Ausgangstemperatur, auf 50 bis 160°C erhöht wird,
d) worauf der entsprechende 2-Acetylcarbonsäureester durch Destillation aus dem Reaktionsgemisch isoliert wird.

Bei dem erfindungsgemäßen Verfahren werden gemäß Schritt a) ein Acetessigsäureester und ein Aldehyd kondensiert. Geeignete Acetessigsäureester sind dabei solche Ester, die 1 bis 6 C-Atome im Alkylteil aufweisen. Beispiele dafür sind Methyl-, Ethyl-, Propyl-, iso-Propyl-, n-Butyl-, Pentyl- oder Hexylester der Acetessigsäure. Bevorzugt werden C₁ bis C₃-Ester eingesetzt., Als Aldehyde werden aliphatische Aldehyde mit 1 bis 12 C-Atomen verwendet. Aliphatische Aldehyde sind dabei geradkettige, verzweigte oder cyclische Aldehyde, die gesättigt aber auch ein- oder mehrfach ungesättigt sein können. Wird ein ungesättigter Aldehyd eingesetzt, ist die beachten, daß die Mehrfachbindungen bei der nachfolgenden Hydrierung reduziert werden. Der Aldehyd kann weiters unsubstituiert oder durch unter den Reaktionsbedingungen inerte Gruppen, wie etwa Phenyl-, substituierte Phenyl-, Halogen-, Nitro-, Alkoxygruppen oder Heterocyclen substituiert sein.

Beispiele dafür sind Formaldehyd als solcher, oder in einer polymeren Form, Acetaldehyd, n-Butyraldehyd, Isobutyraldehyd, Propionaldehyd, Valeraldehyd, Capronaldehyd, Octanal, Dodecanal, Decanal oder 2-Propenal.
Bevorzugt werden aliphatische gesättigte Aldehyde mit 2 bis 10 C-Atomen, besonders bevorzugt mit 3 bis 8 C-Atomen verwendet.
Aldehyd und Ester werden bei dem erfindungsgemäßen Verfahren vorzugsweise äquimolar oder mit dem Ester im Überschuß umgesetzt, da ein Überschuß an Aldehyd zu Aldoladditionen führen kann.

Die Kondensation erfolgt in Anwesenheit eines Kondensationskatalysators. Hierfür eignen sich alle Katalysatoren, die üblicherweise für Kondensationsreaktionen von Aldehyden verwendet werden.
Beispiele sind basische Katalysatoren, wie etwa Ammoniak oder Amine, beispielsweise Piperidin, Pyridin, substituierte Pyridine, Morpholin, Diethylamin, Diethanolamin die gegebenenfalls in Form von Salzen mit organischen Säuren wie Essigsäure, Buttersäure oder Oxalsäure vorliegen können, wie etwa Piperidinacetat.

Andere geeignete Katalysatoren sind beispielsweise andere basische Salze wie etwa Natriumacetat oder basische lonenaustauschharze und saure Katalysatoren wie Zinkacetat, Magnesiumchlorid.
Bevorzugt werden Amine oder deren Salze als Katalysator eingesetzt. Besonders bevorzugt wird Piperidin, Piperidinacetat, Pyridin oder Morpholin verwendet.
Die erforderliche Menge an Katalysator hängt vom Katalysatortyp ab. Basische Katalysatoren werden erfindungsgemäß in Mengen zwischen 0,1 und 3,0 Mol%, bevorzugt zwischen 0,2 und 2 Mol % eingesetzt, während saure Katalysatoren vorzugsweise in etwas größeren Mengen zugesetzt werden.
Gegebenenfalls kann der Katalysator in Kombination mit einem Verdünnungsmittel wie etwa Methanol, Ethanol oder Essigsäure eingesetzt werden.
Die Kondensationsreaktion erfolgt bei Temperaturen von 0 bis 40°C, bevorzugt bei 0 bis 20°C.

Die Reihenfolge der Dosierung der Edukte und des Katalysators kann beliebig gewählt werden.
Bevorzugt wird der Ester und der Aldehyd vorgelegt und der Katalysator langsam zudosiert. Da die Reaktion zu Beginn äußerst rasch und stark exotherm verläuft, ist das Reaktionsgemisch ausreichend zu kühlen, sodaß die Reaktionstemperatur nicht auf über 40°C, bevorzugt nicht auf über 20°C steigt.
Nach Beendigung der Dosierung wird gegebenenfalls noch 10 bis 120 Minuten, bevorzugt 30 bis 90 Minuten bei 0 bis 40°C bevorzugt bei 10 bis 20°C zur Vervollständigung der Kondensation gerührt.

Anschließend werden gemäß Schritt b) das gebildete Reaktionswasser und überschüssige, nicht umgesetzte Edukt aus dem Reaktionsgemisch entfernt.
Bei diesem Reaktionsschritt ist es wesentlich, daß die Temperatur in der Reaktionsapparatur möglichst niedrig gehalten wird, um unerwünschte Nebenreaktionen zu vermeiden. Am schonendsten wird das Reaktionswasser für den Fall, daß die Edukte wasserunlöslich sind, mittels Phasentrennung bei 15 bis 35°C, bevorzugt bei Raumtemperatur, vom Reaktionsgemisch abgetrennt. Die restlichen Wasserspuren können nach der Phasentrennung, gemeinsam mit den nicht umgesetzten Edukten möglichst schonend abdestilliert werden. Das Wasser kann jedoch auch gleich, ohne vorherige Phasentrennung, gemeinsam mit den nicht, umgesetzten Edukten abdestilliert werden. Möglichst schonende Destillationsbedingungen werden bei kurzer Verweilzeit, bevorzugt unter 1 Minute erreicht. Bevorzugte Methoden sind dabei Kurzweg- oder Dünnschichtverdampfer. Besonders bevorzugt werden das Reaktionswasser und überschüssige Edukte kontinuierlich aus dem Reaktionsgemisch abgezogen.

Das erhaltene Destillat bildet zwei Phasen, sodaß die Edukte leicht vom Reaktionswasser abgetrennt und wieder eingesetzt werden können.

Der nach Abtrennung des Reaktionswassers und der überschüssigen Edukte verbleibende Sumpf enthält den entsprechenden 2-Acetylalkensäureester, der in Schritt c) hydriert wird. Dazu wird der Sumpf in einer geeigneten Apparatur, vorgelegt, mit einem Hydrierkatalysator versetzt und bei 20 bis 160°C und einem Druck von 1 bis 100 bar hydriert. Bevorzugt wird bei 5 bis 30 bar hydriert.
Als Hydrierkatalysatoren kommen übliche Hydrierkatalysatoren wie Palladium, Platin, Rhodium, Kobalt oder Ruthenium auf Siliciumdioxyd, auf Aluminiumoxyd oder auf Kohlenstoff, sowie Raney-Nickel-Katalysatoren in Frage. Bevorzugt wird Palladium auf Aluminiumoxyd oder auf Kohlenstoff verwendet. Der Hydrierkatalysator wird dabei in einer Menge von 0,05 bis 0,5 Gew.% bezogen auf 2-Acetyl-2-alkensäureester zugegesetzt.

In der ersten Phase wird die Hydrierung bevorzugt bei niedrigen Temperaturen, bei 20 bis 90°C, durchgeführt. Nach etwa 5 bis 120 Minuten, bevorzugt nach 10 bis 80 Minuten, wird sodann die Temperatur langsam erhöht und das Reaktionsgemisch einer Nachhydrierung unterzogen.

Die Temperatur wird dabei, je nach Ausgangstemperatur in der ersten Hydrierphase, auf 50 bis 160°C, bevorzugt auf 80 bis 150°C gesteigert. Das Reaktionsgemisch wird in dieser Zeit, bis zur Beendigung der Wasserstoffaufnahme weitergerührt. Durch diese zweite Hydrierphase wird gewährleistet, daß Nebenprodukte, die sich durch Michael-Addition gebildet haben, thermisch wieder rückgespalten werden.
Nach dem Hydrierschritt wird das so erhaltene Reaktionsgemisch auf Raumtemperatur abgekühlt, der Katalysator abfiltriert und der entsprechende Ester destillativ aus dem Gemisch isoliert.

Durch das erfindungsgemäße Verfahren können die entsprechenden 2-Acetylcarbonsäureester ohne weitere Reinigung in hohen Ausbeuten von über 80 Gew.% und einer Reinheit von über 98 % hergestellt werden.

Die gemäß vorliegendem Verfahren erzeugten 2-Acetylcarbonsäureester zeichnen sich dabei durch einen vernachlässigbar kleinen Gehalt an Verunreinigungen wie Kondensationsprodukte und thermische Zersetzungsprodukte aus, sodaß sich diese Produkte beispielsweise ausgezeichnet zur Herstellung von Oxetanonen, die als Pankreaslipaseinhibitoren Verwendung finden, sowie zur Herstellung von fungiziden oder insektiziden Pyrimidinverbindungen oder zur Herstellung von Parfumprodukten eignen.

### Beispiel 1:

In einem 2l-Reaktionsgefäß wurden 696,8 g (6,0 mol) Acetessigsäuremethylester und 601,0 g (6,0 mol) Hexanal vorgelegt, wobei das Gemisch auf 5°C abgekühlt wurde. Dazu wurden 6,5 g (0,076 mol) Piperidin unter Kühlen und Rühren innerhalb von 15 Minuten dosiert. Anschließend wurde das gebildete Reaktionswasser, sowie nicht abreagiertes Hexanal und Acetessigester über eine Destillationsbrücke bei 20 mbar (Sumpftemperatur: 20 - 150°C) abdestilliert.
Dabei wurden 1059,7 g Acetyloctensäuremethylester als Sumpfprodukt und 244,2 g nicht reagierte Edukte sowie Reaktionswasser im Destillat erhalten. 330 g des oben hergestellen Acetyl-2-octensäuremethylesters (Sumpfprodukt) wurden sodann in einem 1-Liter-Rühr-Autoklaven vorgelegt, mit 0,33 g 5 % Palladium/Aktivkohle als Katalysator versetzt und bei 90°C und 15 bar 10 Minuten hydriert.
Anschließend wurde das Reaktionsgemisch langsam auf 150°C erhitzt und bis zur Beendigung der Wasserstoffaufnahme bei dieser Temperatur gerührt.
Nach der Hydrierung wurde das Reaktionsgemisch auf Raumtemperatur abgekühlt, der Katalysator abfiltriert und über eine Glockenbodenkolonne (6 Böden, Durchmesser 3 cm, Länge 35 cm) bei 5 mbar destilliert.
Der Vorlauf wurde bei einem Rücklaufverhältnis von 5 : 1 (5 Rücklauf/1 Destillat), der Hauptlauf bei einem Rücklaufverhältnis von 3 : 1 abgenommen.

| | |
|---|---|
| Vorlauf | (22 - 121°C) 36,1 g |
| Hauptfraktion | (120-123°C) |
| Ausbeute | 240,9 g 2-Acetyloctansäuremethylester ( = 64,5 % der Theorie) |
| Reinheit (GC) | 98,2 % |
| Sumpfrückstand | 41,9 g |

Die abdestillierten, nichtreagierten Edukte wurden nach Abtrennen des Reaktionswassers für einen weiteren Reaktionsansatz rückgeführt und wiedereingesetzt, wodurch eine Ausbeutesteigerung auf 76 % erzielt wurde.

### Beispiel 2:

Es wurden insgesamt 7 Chargen nach der gleichen Fahrweise produziert. Dabei wurden jeweils Acetessigsäuremethylester (AEE) und Hexanal (HEX) vorgelegt und mit Sole auf ca. 8°C gekühlt. Anschließend wurde innerhalb von 2 Minuten die gesamte Katalysatormenge (Piperidin PIP) zugegeben. Der Reaktor wurde während der gesamten Reaktion mit Sole gekühlt. Die Reaktortemperatur stieg nach der Katalysatorzugabe innerhalb von 5 Minuten auf einen Maximalwert zwischen 30 und 40°C an und fiel aufgrund der Solekühlung innerhalb einer Stunde auf 14°C wieder ab.
Die genauen Chargiermengen und Temperaturwerte sind aus Tabelle 1 ersichtlich.
Bei den Chargen 4 bis 7 wurde als Einsatzstoff zusätzlich ein Recyclat eingesetzt. Das Recyclat wurde durch Abdestillieren der unumgesetzten Edukte nach erfolgter Kondensation von der jeweiligen Vorcharge erhalten.

**Tabelle 1**

| Charge | AEE (kg) | HEX (kg) | PIP (l) | Recyclat (kg) | (°C) | |
|---|---|---|---|---|---|---|
| | | | | | Start | max. |
| 1 | 3487 | 3274 | 37 | - | 16 | 39 |
| 2 | 3488 | 3273 | 30 | - | 11 | 37 |
| 3 | 3486 | 3266 | 30 | - | 11 | 35 |
| 4 | 1420 | - | 20 | 4740 | 10 | 25 |
| 5 | 1678 | 291 | 20 | 5580 | 10 | 27 |
| 6 | 2027 | 1489 | 20 | 2973 | 10 | 30 |
| 7 | 1597 | 667 | 20 | 3000 | 9 | 36 |

Nach einer Reaktionszeit von 5 Minuten wurde bereits ein 2-Acetylactensäuremethylester (ACOEM) Gehalt von 50 - 60 % mit 0,1 bis 0,2 Gew. % Kondensationsprodukt ermittelt.
Nach ca. 6 Stunden Reaktionszeit wurden jeweils etwa 60 - 70 Gew.% ACOEM mit 0,4 - 0,6 Gew.% Kondensationsprodukt erhalten.
Die nicht umgesetzten Edukte und das Reaktionswasser wurden am Kurzwegverdampfer abdestilliert.

| | |
|---|---|
| Produktzufuhr | 500 I/h |
| Heizdampfdruck | 2 bar |
| Vakuum | 40 mbar |
| Sumpfanteil | 60 % |

### Ergebnis Produktqualität in GC-Flächenprozent

| | ACOEM | AEE | HEX | Kond.Prod. |
|---|---|---|---|---|
| Kopf | 10-20 | 20-25 | 50-60 | 0 |
| Sumpf | 91-93 | 1-3 | 1-2 | 1 - 1,5 |
| Kond.Prod......Kondensationsprodukt | | | | |

Für die Hydrierung wurde ein 5 % Pd/C Katalysator K-0227 der Fa. Heraeus eingesetzt, der nach erfolgter Hydrierung über zwei Katalysator-Filter abfiltriert und für die jeweils nächste Charge eingesetzt wurde. Es wurden insgesamt 5 kg Katalysator eingesetzt, die am Ende der Kampagne noch voll aktiv waren.

| Hydrierbedingungen: | |
|---|---|
| 1. Phase Hydrierung | 80°C bis kein H₂ mehr aufgenommen wurde. |
| 2. Phase Hydrierung | 150°C 1 Stunde |
| Hydrierdruck | 10 bar |
| ACEOM Chargiermenge | 5000 kg |

Der nach Abfiltrieren des Katalysators erhaltene 2-Acetyloctansäuremethylester wurde anschließend kontinuierlich destilliert.

| | |
|---|---|
| Kopftemperatur | 135 - 145°C |
| Sumpftemperatur | 180 - 185°C |
| Vakuum | 50 - 9 mbar |
| Ausbeuten | (Σ Charge 1-7) 19850 kg (=81 % d.Th. bezogen auf Hexanal) |
| Reinheit | 99,2 % |

### Beispiel

### 1. Ansatz:

In einem 2 Liter Reaktionsgefäß wurden 348,4 g Acetessigsäuremethylester (3,00 mol) und 300,5 g Hexanal (3,00 mol) vorgelegt, wobei das Gemisch auf 5°C abgekühlt wurde. Dazu wurden 1,5 g Piperidin (0,018 mol) unter Kühlen und Rühren innerhalb von 15 Minuten dosiert und weitere 2 Stunden bei 30°C gerührt.
Anschließend wurde das gebildete Reaktionswasser sowie nicht abreagiertes Hexanal und Acetessigester über eine Dünnschichtdestillationsapparatur destilliert.
(Temperatur der Heizung: 130°C, Druck 20 mbar, Destillattemperatur: 82-89°C)

Es wurden 367,1 g 2-Acetyl-2-octensäuremethylester als Sumpfprodukt und 274,6 g nicht reagierte Einsatzstoffe sowie Reaktionswasser im Destillat erhalten.

Das Sumpfprodukt (2-Acetyl-2-octensäuremethylester) wurde in einem 1 Liter Rühr-Autoklaven vorgelegt, mit 0,3 g 5 % Palladium/Aktivkohle als Katalysator versetzt und bei 90°C 10 Minuten hydriert.

Anschließend wurde das Reaktionsgemisch auf Raumtemperatur abgekühlt, der Katalysator abfiltriert und über eine Vigreuxkolonne (Höhe 15 cm, Durchmesser: 3 cm) mit Destillationsbrücke bei 1 mbar destilliert.

| | |
|---|---|
| Vorlauf /79 - 82°C) | 7,8 g |
| Hauptfraktion (82 - 90°C) | 340,1 g (= 56,7 % d. Th.) 97,9 FI% (GC) |
| Sumpf | 18,6 g |

### 2. Ansatz:

Die Reaktion und das Abdestillieren der nicht reagierten Edukte und des Reaktionswassers wurde analog zu 1. Ansatz durchgeführt.

Es wurden 370,6 g Acetyloctensäuremethylester als Sumpfprodukt und 277,2 g nicht reagierte Einsatzstoffe sowie Reaktionswasser im Destillat erhalten.

Die Hydrierung und Abtrennung des Katalysators sowie die Destillation wurde analog zu Ansatz 1 durchgeführt.

| Mengen: | |
|---|---|
| Vorlauf /79 - 82°C) | 10,2 g |
| Hauptfraktion /82 - 90°C) | 335,5 g (55,9 % d.Th.) 98,2 FI% (GC) |
| Sumpf | 16,9 g |

### 3. Ansatz

Die Reaktion und das Abdestillieren der nicht reagierten Edukte und des Reaktionswassers wurde analog zu 1. Ansatz durchgeführt.

Es wurden 384,2 g Acetyloctensäuremethylester als Sumpfprodukt und 257,8 g nicht reagierte Einsatzstoffe sowie Reaktionswasser im Destillat erhalten.

Die Hydrierung und Abtrennung des Katalysators sowie die Destillation wurde analog zu Ansatz 1 durchgeführt.

| | |
|---|---|
| Vorlauf (79 - 82°C) | 6,5 g |
| Hauptfraktion (82-90°C) | 345,8 g (57,6 % d. Th.) 98,4 FI% (GC) |
| Sumpf | 18,3 g |

### 4. Ansatz (Einsatz der abdestillierten nicht reagierten Edukte aus Ansatz 1 - 3 nach Abtrennung der Wasserphase durch Phasentrennung).

In einem 2 Liter Reaktionsgefäß wurden 235,0 g organische Phase aus Destillat (Ansatz 1) + 236,6 g organische Phase aus Destillat (Ansatz 2) + 216,5 g organische Phase aus Destillat (Ansatz 3) vorgelegt, wobei das Gemisch auf 5°C abgekühlt wurde. Dazu wurden 1,6 g Piperidin (0,019 mol) unter Kühlen und Rühren innerhalb von 15 Minuten dosiert und weitere 2 Stunden bei 30°C gerührt.
Anschließend wurde das gebildete Reaktionswasser sowie nicht abreagiertes Hexanal und Acetessigester über eine Dünnschichtdestillatonsapparatur destilliert.
(Temperatur der Heizung: 130°C, Druck 20 mbar, Destillattemperatur: 82 - 89°C)

Es wurden 480,5 g Acetyloctensäuremethylester als Sumpfprodukt und 198,9 g nicht reagierte Einsatzstoffe sowie Reaktionswasser im Destillat erhalten.

Die Hydrierung und die Abtrennung des Katalysators sowie die Destillation wurde analog zu Ansatz 1 durchgeführt.

| | |
|---|---|
| Vorlauf (79 - 82°C) | 13,6 g |
| Hauptfraktion (82 - 90°C) | 429,5 g 98,2 FI% (GC) |
| Sumpf | 31,4 g |
| Ausbeute (Ansatz 1 - 4) | 1451 g (= 80,6 % d.Th.) |
| Reinheit (GC-FI%) | 98,1 % |

## Patentansprüche

1. Verfahren zur Herstellung von 2-Acetylcarbonsäureester durch Reaktion eines Acetessigsäureesters mit einem Aldehyd und anschließender Hydrierung, dadurch gekennzeichnet, daß
a) ein Acetessigsäureester mit 1 bis 6 C-Atomen im Alkylteil mit einem aliphatischen Aldehyd mit 1 bis 12 C-Atomen in Gegenwart eines Kondensationskatalysators bei 0 bis 40°C umgesetzt wird,
b) nach erfolgter Kondensation das gebildete Reaktionswasser, sowie nicht umgesetzte Edukte aus dem Reaktionsgemisch entfernt werden, anschließend
c) der verbleibende Rückstand in Gegenwart eines Hydrierkatalysators in Substanz bei 20 bis 160°C und einem Druck von 1 bis 100 bar hydriert wird, wobei in der ersten Phase des Hydrierens die Temperatur zwischen 20 und 90 °C liegt und in der zweiten Phase die Temperatur, je nach Ausgangstemperatur, auf 50 bis 160°C erhöht wird,
d) worauf der entsprechende 2-Acetylcarbonsäureester durch Destillation aus dem Reaktionsgemisch isoliert wird.

2. Verfahren nach Anspruch 1, dadurch gekennzeichnet, daß ein Acetessigester mit 1 bis 3 C-Atomen im Alkylteil eingesetzt wird.

3. Verfahren nach Anspruch 1, dadurch gekennzeichnet, daß als Aldehyd ein unsubstituierter oder durch unter den Reaktionsbedingungen inerte Gruppen substituierter, geradkettiger, verzweigter oder cyclischer Aldehyd, der gesättigt oder ein- oder mehrfach ungesättigt sein kann, eingesetzt wird.

4. Verfahren nach Anspruch 2, dadurch gekennzeichnet, daß als Aldehyd ein aliphatischer, gesättigter Aldehyd mit 2 bis 10 C-Atomen eingesetzt wird.

5. Verfahren nach Anspruch 1, dadurch gekennzeichnet, daß als Kondensationskatalysator Ammoniak, Piperidin, Pyridin, substituierte Pyridine, Morpholin, Diethylamin, Diethanolamin oder deren Salze mit organischen Säuren eingesetzt werden.

6. Verfahren nach Anspruch 1, dadurch gekennzeichnet, daß das Reaktionswasser und nicht umgesetzte Edukte kontinuierlich abdestilliert werden.

7. Verfahren nach Anspruch 1, dadurch gekennzeichnet, daß Schritt c) bei 5 bis 30 bar erfolgt.

8. Verfahren nach Anspruch 1, dadurch gekennzeichnet, daß die zweite Hydrierphase bei 80 bis 150°C erfolgt.

## Claims

1. Process for the preparation of a 2-acetylcarboxylic ester by reacting an acetoacetic ester with an aldehyde, followed by hydrogenation, characterized in that
a) an acetoacetic ester having 1 to 6 C atoms in the alkyl moiety is reacted with an aliphatic aldehyde having 1 to 12 C atoms at 0 to 40°C in the presence of a condensation catalyst,
b) after condensation, the water of reaction formed and unreacted starting materials are removed from the reaction mixture, subsequently
c) the remaining solid residue is hydrogenated at 20 to 160°C and a pressure of 1 to 100 bar in the presence of a hydrogenation catalyst, the temperature in the first hydrogenation phase being between 20 and 90°C and the temperature in the second phase being increased to 50 to 160°C, depending on the starting temperature,
d) and subsequently the 2-acetyl-carboxylic ester in question is isolated from the reaction mixture by distillation.

2. Process according to Claim 1, characterized in that an acetoacetic ester having 1 to 3 C atoms in the alkyl moiety is employed.

3. Process according to Claim 1, characterized in that the aldehyde employed is a straight-chain, branched or cyclic aldehyde which can be saturated or mono- or polyunsaturated and which is unsubstituted or substituted by groups which are inert under the reaction conditions.

4. Process according to Claim 2, characterized in that the aldehyde employed is an aliphatic saturated aldehyde having 2 to 10 C atoms.

5. Process according to Claim 1, characterized in that the condensation catalyst employed is ammonia, piperidine, pyridine, substituted pyridines, morpholine, diethylamine, diethanolamine or salts of these with organic acids.

6. Process according to Claim 1, characterized in that the water of reaction and unreacted starting materials are distilled off continuously.

7. Process according to Claim 1, characterized in that step c) is carried out at 5 to 30 bar.

8. Process according to Claim 1, characterized in that the second hydrogenation phase is carried out at 80 to 150°C.

## Revendications

1. Procédé pour fabriquer des esters de l'acide 2-acétylcarboxylique par réaction d'un ester de l'acide acétylacétique avec un aldéhyde et hydrogénation ultérieure, caractérisé en ce que
a) on convertit un ester d'acide acétylacétique contenant 1 à 6 atomes de C dans la partie alkyle avec un aldéhyde aliphatique contenant 1 à 12 atomes de C en présence d'un catalyseur de condensation, entre 0 et 40°C,
b) une fois réalisée la condensation, on élimine du mélange réactionnel l'eau de réaction formée avec des produits éduits non transformés, puis
c) on hydrogène le résidu restant en présence d'un catalyseur d'hydrogénation en substance entre 20 et 160°C et sous une pression de 1 à 100 bars, auquel cas dans la première phase de l'hydrogénation, la température est comprise entre 20 et 90°C et dans la seconde phase, la température est accrue entre 50 et 160°C, en fonction de la température de départ,
d) à la suite de quoi on isole l'ester d'acide 2-acétylcarboxylique correspondant par distillation à partir du mélange réactionnel.

2. Procédé selon la revendication 1, caractérisé en ce qu'on utilise un ester d'acide acétylacétique contenant 1 à 3 atomes de C dans la partie alkyle.

3. Procédé selon la revendication 1, caractérisé en ce qu'on utilise comme aldéhyde un aldéhyde non substitué ou substitué par des groupes inertes dans les conditions de réaction, à chaîne droite, ramifié ou cyclique qui peut être saturé ou non saturé selon un mode simple ou multiple.

4. Procédé selon la revendication 2, caractérisé en ce qu'on utilise comme aldéhyde un aldéhyde aliphatique saturé contenant 2 à 10 atomes de C.

5. Procédé selon la revendication 1, caractérisé en ce qu'on utilise comme catalyseur de condensation, de l'ammoniac, de la pipéridine, de la pyridine, de la pyridine substituée, de la morpholine, de la diéthylamine, de la diéthanolamine ou leurs sels avec des acides organiques.

6. Procédé selon la revendication 1, caractérisé en ce que l'eau de réaction et des produits éduits non transformés sont séparés en continu par distillation.

7. Procédé selon la revendication 1, caractérisé en ce que l'étape c) s'effectue à une pression comprise entre 5 et 30 bars.

8. Procédé selon la revendication 1, caractérisé en ce que la seconde phase d'hydrogénation s'effectue entre 80 et 150°C.
